# DEMANDE DE BREVET EUROPEEN

(11) **EP 1 316 301 A1**
(43) Date de publication de la demande: **04.06.2003**
(21) Numéro de dépôt: 02292680.2
(22) Date de dépôt: 29.10.2002
(51) Int. Cl.: A61K 7/48, A61F 2/10, A61L 27/60

(54) **Composition cosmétique ou dermatologique comprenant un rétinoide et/ou un caroténoide et l'acide acexamique.**

(30) Priorité: 30.11.2001 FR 0115518
(71) Demandeur: L'OREAL, 75008 Paris (FR)
(72) Inventeur: Duranton, Albert, 78600 Maison Laffite (FR); Renault, Béatrice, 94410 Saint Maurice (FR); Mahe, Yann, 91390 Morsang sur Orge (FR); Marion, Catherine, 92160 Antony (FR); Catroux, Philippe, 94130 Nogent sur Marne (FR)
(74) Mandataire: Renard, Emmanuelle

(57) **Abrégé**

L'invention se rapporte à l'utilisation cosmétique d'une composition comprenant, dans un milieu physiologiquement acceptable, de 0,001% à 1% en poids d'acide acéxamique et/ou l'un de ses sels, et de 0,00001% à 0,1% en poids d'au moins un composé choisi parmi les rétinoïdes et les caroténoïdes pour prévenir ou lutter contre les gerçures, les tiraillements et/ou les rougeurs.

Elle a aussi pour objet l'utilisation d'au moins un premier composé choisi parmi l'acide acexamique et ses sels, et d'au moins un second composé choisi parmi les rétinoïdes et caroténoïdes, pour fabriquer respectivement une première et une seconde préparations dermatologiques destinées à être administrées successivement pour favoriser la ré épithélialisation de la peau ou des muqueuses.

## Description

L'invention se rapporte à l'utilisation cosmétique d'une composition comprenant, dans un milieu physiologiquement acceptable, de 0,001% à 1% en poids d'acide acéxamique et/ou l'un de ses sels, et de 0,00001% à 0,1% en poids d'au moins un composé choisi parmi les rétinoïdes et les caroténoïdes pour prévenir ou lutter contre les gerçures, les tiraillements et/ou les rougeurs.

Elle concerne aussi les utilisations dermatologiques de cette composition, en particulier pour favoriser la ré-épithélialisation de la peau ou des muqueuses.

Les agressions de l'environnement, en particulier les variations de température et le contact avec des substances chimiques irritantes (telles que les détergents), provoquent un dessèchement de la peau et des muqueuses responsable de sensations d'inconfort, de tiraillements, de démangeaisons, de rougeurs, voire de gerçures ou de crevasses.

Cela est en particulier vrai pour les surfaces du corps humain particulièrement exposées, et par ailleurs fragiles, que sont les lèvres et les mains, ces dernières étant constituées d'une peau très fine. Par ailleurs, ces sensations désagréables sont plus marquées chez les personnes stressées ou encore chez les personnes âgées dont la barrière cutanée est affaiblie, notamment en raison d'une réduction des biosynthèses épidermiques au cours du vieillissement.

Les désordres cutanés résultant de ces agressions de l'environnement sont dus à une altération, voire une rupture, dans la continuité de la couverture dermique par les cellules épidermiques formant normalement un "tapis" protecteur continu et pluristratifié.

On comprend donc l'utilité de disposer d'une composition permettant de reconstituer ou de préserver ce "tapis protecteur", c'est-à-dire de permettre la ré-épithélialisation des tissus.

En dehors des sujets sains, une telle composition présenterait également un intérêt pour les personnes souffrant d'altérations traumatiques telles que des plaies étendues et des brûlures. Dans le cas des grands brûlés, notamment, un facteur limitant le pronostic vital est qu'une surface de peau minimale doit conserver, au mieux, les caractéristiques d'une peau dite normale quant au nombre de couches cellulaires épidermiques et à l'intégrité de la fonction barrière.

Différentes solutions ont été envisagées jusqu'à présent pour prévenir ou traiter les désordres cutanés mentionnés précédemment.

Ainsi, les crèmes pour les mains disponibles dans le commerce contiennent habituellement des composés destinés à protéger la peau en formant un film protecteur sur celle-ci (vaseline, collagène, cires, huiles végétales, huiles de silicone), en l'humectant (allantoïne, urée, glycérol, sorbitol, lactate d'ammonium), en l'apaisant ou en favorisant la cicatrisation (aloès, bisabolol, vitamines A , E, F et B5, allantoïne), en filtrant les rayonnements UVA et/ou UVB, et/ou en la nourrissant (acides gras essentiels, beurre de karité).

Ces crèmes présentent toutefois une efficacité parfois insuffisante et ont surtout l'inconvénient d'offrir un toucher gras et collant peu cosmétique.

En outre, il a déjà été proposé dans la demande de brevet EP-0 940 137 d'utiliser l'acide acexamique [appelé aussi l'acide acétamido-caproïque ou encore l'acide 6-(acétylamino) hexanoïque] sous forme libre ou de sel de zinc dans une composition pour la protection et le soin des lèvres, en particulier contre les gerçures. Il a également été suggéré dans la publication de Guillard et al., Pharmacology 34 : 296-300 (1987) d'utiliser l'acide acexamique dans le traitement des plaies et pour favoriser la cicatrisation. Toutefois, la Demanderesse a mis en évidence que si l'acide acexamique permettait effectivement la formation d'un nouvel épithélium in vitro, les couches épithéliales formées ne sont pas de très bonne qualité, puisque fusiformes et non jointives.

On sait par ailleurs que les rétinoïdes, dont le rétinol, sont capables d'accélérer la ré-épithélialisation des peaux lésées en activant la prolifération épidermique (M. Verschoore et al., Topical Retinoids - Their Uses in Dermatology, Dermatologic Clinics, Vol. 11, No. 1, Janvier 1993). Toutefois, la Demanderesse a démontré que l'épiderme ainsi obtenu n'était pas suffisamment épais et bien différencié.

Il reste donc le besoin de disposer d'une composition à usage cosmétique ou dermatologique, permettant d'obtenir une ré-épithélialisation satisfaisante de la peau ou des muqueuses, tant sur le plan quantitatif que qualitatif, tout en offrant un toucher cosmétiquement acceptable.

Or, la Demanderesse a découvert que l'utilisation conjointe ou successive d'acide acexamique (ou l'un de ses sels) et d'au moins un composé choisi parmi un rétinoïde et un caroténoïde permettait de satisfaire ce besoin.

Il était, certes, déjà connu de CUCIUREANU et al., Caracterisation physico-chimique des onguents lipophiles avec vitamine A, Congr. Int. Technol. Pharm., Vol. 4, pages 196-202 (1992) d'introduire l'acide acexamique, en tant qu'agent anti-oxydant, dans des préparations lipophiles à base de rétinoïdes tels que l'acétate de rétinol, le rétinal ou l'acide rétinoïque, en vue de limiter la dégradation de ces composés. Toutefois, ce document ne suggère pas que la préparation ainsi stabilisée puisse avoir un quelconque effet de ré-épithélialisation et ne mentionne que l'effet bien connu des rétinoïdes dans le traitement des signes du vieillissement actinique.

La présente invention a donc pour objet l'utilisation cosmétique d'une composition comprenant, dans un milieu physiologiquement acceptable, de 0,001% à 1% en poids d'acide acéxamique et/ou l'un de ses sels, et de 0,00001% à 0,1% en poids d'au moins un composé choisi parmi les rétinoïdes et les caroténoïdes pour prévenir ou lutter contre les gerçures, les tiraillements et/ou les rougeurs.

Cette composition est ainsi avantageusement appliquée sur les mains, en particulier les mains desséchées et/ou abîmées. En variante, elle peut être appliquée sur les lèvres.

La composition selon l'invention protège la peau ou les muqueuses contre les agressions de l'environnement. Elle permet d'obtenir un résultat durable sans offrir de toucher gras ni collant.

La Demanderesse a en effet découvert que l'application conjointe ou séquentielle d'acide acexamique ou l'un de ses sels, puis d'un rétinoïde ou caroténoïde, avait l'avantage non seulement de permettre la potentialisation par l'acide acexamique de l'effet du rétinoïde ou caroténoïde sur la prolifération et la différenciation des cellules épithéliales, mais aussi de contrer les effets, négatifs à long terme, de l'acide acexamique sur la différenciation de l'épithélium.

Les rétinoïdes utilisables dans la composition selon l'invention peuvent être choisis parmi : le rétinol, le rétinal, l'acide rétinoïque 13-cis, l'acide rétinoïque all-trans et les esters de rétinyle tels que l'acétate de rétinyle, le propionate de rétinyle et le palmitate de rétinyle. Le palmitate de rétinyle est préféré pour une utilisation dans la présente invention.

Comme caroténoïdes, on peut citer ceux ayant ou non une activité vitaminique A, dont certains sont des précurseurs de rétinoïdes, et en particulier l'α-carotène, le β-carotène, le lycopène, la zéaxanthine, la lutéine, l'ataxanthine, la canthaxanthine et la cryptoxanthine.

Le caroténoïde utilisé selon l'invention peut être d'origine naturelle ou synthétique. Par origine naturelle, on entend le caroténoïde, à l'état pur ou en solution quelle qu'en soit sa concentration dans ladite solution, obtenu à partir d'un élément naturel. Par origine synthétique, on entend le caroténoïde, à l'état pur ou en solution quelle qu'en soit sa concentration dans ladite solution, obtenu par synthèse chimique.

Lorsque le caroténoïde est d'origine naturelle, il peut être obtenu à partir de matériel végétal issu de plante entière cultivée *in vivo* ou issu de culture *in vitro*. Par culture *in vivo* on entend toute culture de type classique c'est à dire en sol à l'air libre ou en serre, ou encore hors sol. Par culture *in vitro*, on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo*.

Préférentiellement selon l'invention, on utilise un végétal issu de culture *in vivo*.

Les caroténoïdes préférés selon l'invention sont le β-carotène et le lycopène.

Le lycopène est un pigment naturel que l'on trouve dans les fruits mûrs, particulièrement dans la tomate. Sa structure est proche de celle du β-carotène. Il peut être sous forme cis ou trans.

A titre d'exemple, selon l'invention on utilise un extrait de tomate riche en lycopène, préparé par la société Métaphar, commercialisé sous la dénomination LycOMato® constitué d'un extrait d'oléorésine (phase grasse) contenant 6% de lycopène pur.

Comme sels d'acide acexamique, on peut citer les sels organiques et inorganiques présentant de préférence un effet activateur de l'urokinase (qui est une enzyme intervenant, en particulier chez les sujets âgés ou stressés, dans la régénération de l'épiderme mais aussi dans les phénomènes de ré-épithélialisation et de cicatrisation) et en particulier les sels de métaux alcalino-terreux et les sels de métaux de transition de l'acide acexamique, tels que les sels de zinc, de calcium et/ou de magnésium d'acide acexamique.

On utilise de préférence l'acide acexamique libre, avantageusement sous forme de poudre, commercialisé par la société SANOFI CHIMIE sous la dénomination commerciale Acide Acexamique N.

La composition selon l'invention renferme généralement une quantité efficace de rétinoïde ou caroténoïde et d'acide acexamique pour obtenir l'effet recherché, c'est-à-dire de 0,001% à 1% en poids, mieux, de 0,01% à 0,1% en poids, d'acide acexamique et/ou de son sel, et de 0,00001% à 0,1% en poids, mieux, de 0,001% à 0,01% en poids, de rétinoïde ou caroténoïde, par rapport au poids total de la composition.

La composition selon l'invention est avantageusement adaptée à une application topique sur la peau ou les muqueuses. Par "peau", on entend aussi bien la peau du visage que celle du corps, en particulier des mains. Par "muqueuses", on entend en particulier les lèvres. Par "milieu physiologiquement acceptable", on entend donc un milieu compatible avec la peau et/ou les muqueuses.

Outre ses applications cosmétiques, l'association d'acide acexamique et de rétinoïde ou caroténoïde selon la présente invention trouve également un intérêt dans le domaine dermatologique. Dans ce domaine, il sera avantageux d'utiliser au moins un premier composé choisi parmi l'acide acexamique et ses sels, et au moins un second composé choisi parmi les rétinoïdes et caroténoïdes, pour fabriquer respectivement une première et une seconde préparations dermatologiques destinées à être administrées successivement pour favoriser la ré-épithélialisation de la peau ou des muqueuses.

L'invention a donc également pour objet l'utilisation d'au moins un premier composé choisi parmi l'acide acexamique et ses sels, et d'au moins un second composé choisi parmi les rétinoïdes et caroténoïdes, pour fabriquer respectivement une première et une seconde préparations dermatologiques destinées à être administrées successivement pour favoriser la ré-épithélialisation de la peau ou des muqueuses.

Ces première et seconde préparations peuvent être plus particulièrement destinées à favoriser la cicatrisation des plaies ou brûlures.

Selon une variante de l'invention, la première préparation peut être appliquée topiquement sur la peau ou les muqueuses et la seconde préparation administrée par voie orale ou par voie parentérale. Selon une autre variante, la première préparation et la seconde préparation peuvent être appliquées topiquement sur la peau ou les muqueuses. Selon une autre variante encore, la première et la seconde préparations peuvent être administrées par voie orale.

L'administration successive des deux composés selon l'invention peut, selon une autre forme d'exécution, être utilisée dans la fabrication d'une peau reconstruite ou artificielle.

L'invention étend donc également sa portée à un procédé de fabrication d'une peau reconstruite ou artificielle, comprenant les étapes successives (a) d'application sur un équivalent dermique d'une première composition comprenant au moins un premier composé choisi parmi l'acide acexamique et ses sels, (b) d'application sur ledit équivalent dermique d'une seconde composition comprenant au moins un second composé choisi parmi les rétinoïdes et caroténoïdes.

Par "équivalent dermique", on entend tout support d'origine biologique (c'est-à-dire d'origine cellulaire, comprenant de préférence des fibroblastes et/ou des cellules endothéliales) et/ou biochimique (c'est-à-dire d'origine protéique, comprenant de préférence du collagène et/ou de la fibronectine, et/ou à base de mucopolysaccharides, comprenant de préférence de l'acide hyaluronique), le support pouvant en outre comprendre une portion synthétique.

La composition et les préparations selon l'invention peuvent se présenter sous toutes les formes galéniques normalement utilisées dans les domaines cosmétique et dermatologique, et elles peuvent être notamment sous forme d'une solution huileuse éventuellement gélifiée, d'une émulsion obtenue par dispersion d'une phase grasse dans une phase aqueuse (H/E) ou inversement (E/H), d'une émulsion triple (E/H/E ou H/E/H), d'une dispersion vésiculaire de type ionique (liposomes ou oléosomes) et/ou non ionique (niosomes) et/ou d'une dispersion de nanocapsules ou nanosphères.

La composition selon l'invention est de préférence sous la forme d'une émulsion eau-dans-huile (E/H).

En outre, le rétinoïde ou caroténoïde utilisé dans la composition selon l'invention est de préférence encapsulé, avantageusement dans des nanocapsules, de façon à retarder sa libération et d'obtenir ainsi un effet séquentiel de l'acide acexamique puis du rétinoïde et/ou du caroténoïde après application de la composition sur la peau. La Demanderesse a en effet mis en évidence, comme cela ressort de l'Exemple 1 ci-après, que l'application séquentielle de ces composés permettait une ré-épithélialisation optimale de la peau.

La composition et les préparations selon l'invention peuvent être plus ou moins fluides et avoir l'aspect d'une crème blanche ou colorée, d'une pommade, d'un lait, d'une lotion, d'un sérum, d'une pâte, d'une mousse. Elle peuvent éventuellement être appliquée sur la peau sous forme d'aérosol ou de patch. Elle peuvent également se présenter sous forme solide, en particulier sous forme de stick pour les lèvres ou de rouge à lèvres. Elle peuvent être utilisées comme produit de soin et/ou comme produit de maquillage pour la peau ou les lèvres et/ou comme produit de rasage.

De façon connue, la composition et les préparations selon l'invention peuvent contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse ou dans la phase aqueuse. En tout état de cause, ces adjuvants, ainsi que leurs proportions, seront choisis de manière à ne pas nuire aux propriétés recherchées de l'association d'actifs selon l'invention.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le co-émulsionnant sont généralement présents, dans la composition, en une proportion allant de 0,3 à 30 % en poids, et de préférence de 0,5 à 20 % en poids par rapport au poids total de la composition.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone et la fraction liquide du beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol; le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Comme émulsionnants et coémulsionnants utilisables dans l'invention, on peut citer par exemple les émulsionnants H/E tels que les esters d'acide gras et de polyéthylène glycol, notamment le stéarate de PEG-100, et les esters d'acide gras et de glycérine tels que le stéarate de glycéryle, ainsi que les émulsionnants E/H tels que le poly(méthylcétyl)(diméthyl)méthylsiloxane oxyéthyléné disponible sous la dénomination commerciale ABIL WE09 auprès de la société Degussa Goldschmidt ou le mélange de stéarate d'éthylène glycol acétyle et de tristéarate de glycéryle commercialisé par la société Guardian sous la dénomination commerciale UNITWIX.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme charges qui peuvent être utilisées dans la composition de l'invention, on peut citer par exemple, outre les pigments, la poudre de silice ; le talc ; l'amidon réticulé par l'anhydride octénylsuccinique commercialisé par la société National Starch sous la dénomination DRY FLO PLUS (28-1160) ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition ou de la préparation selon l'invention.

Enfin, on peut citer comme actifs utilisables dans la composition ou les préparations selon l'invention : le collagène, l'allantoïne, l'urée, le glycérol, le sorbitol, le lactate d'ammonium, les extraits d'aloès, le bisabolol, et les vitamines E, F et B5. En cas d'incompatibilité ou pour les stabiliser, certains au moins des actifs mentionnés ci-dessus peuvent être incorporés dans des sphérules, notamment des vésicules ioniques ou non-ioniques et/ou des nanoparticules (nanocapsules et/ou nanosphères).

On peut également introduire dans la composition ou les préparations selon l'invention des filtres UVA et/ou UVB, choisis parmi les filtres organiques et les filtres minéraux éventuellement enrobés pour les rendre hydrophobes.

Selon une forme de réalisation avantageuse de l'invention, l'acide acexamique peut être compatibilisé dans la phase grasse des compositions ou des préparations selon l'invention, en particulier lorsqu'il s'agit d'une phase anhydre, au moyen d'au moins un polyol liquide à température ambiante, ayant un indice d'IOB compris entre 1 et 7. La Demanderesse a en effet découvert que ces polyols constituaient de très bons solvants de l'acide acexamique.

Le paramètre IOB est connu de l'homme du métier à partir d'un certain nombre de publications comme l'article de A. FUJITA Pharm. Bull 2, 163-173 (1954) et les documents J0 9/151109, J08/217639 ou J09/175925.

A titre d'exemples de polyols qui vérifient les critères précédents, et qui peuvent être utilisés dans les compositions et préparations de l'invention, on peut citer les alkylènes glycols, tels que le propylène glycol, le butylène glycol, le pentylène glycol et l'hexylène glycol ; les polyéthylène glycols (PEG) ayant de 4 à 8 motifs d'éthylène glycol, tels que le PEG-4, le PEG-6 ou le PEG-8 ; le glycérol ; et le panthénol.

Dans le cas où la première et/ou la seconde préparation sont administrées par voie orale, elles peuvent se présenter sous toute forme galénique convenant à ce mode d'administration, par exemple sous forme de comprimés sécables ou non, de granules, de capsules, de gélules, de solutés, de suspensions ou de solutions comprenant un excipient approprié. Des exemples d'excipients convenant à ce mode d'administration comprennent un mélange de dextrose et de cellulose éventuellement renforcés par du stéarate de magnésium comme agent de liaison et lubrifiant.

La première préparation, lorsqu'elle est administrée par voie orale, renferme une quantité suffisante d'acide acexamique ou son sel pour obtenir l'effet recherché, par exemple une quantité permettant l'administration, en une ou plusieurs prises, de 300 à 600 mg par jour d'acide acexamique.

La seconde préparation, lorsqu'elle est administrée par voie orale, renferme une quantité suffisante de rétinoïde ou caroténoïde pour obtenir l'effet recherché, de façon par exemple à apporter une dose de carotène correspondant à 400 µg de vitamine A, soit environ 3 mg de carotène, ou de façon à apporter une dose d'autres caroténoïdes tels que le lycopène, la zéaxanthine, la cryptoxanthine ou la lutéine, correspondant à 1,6 mg par jour.

L'invention sera mieux comprise, et ses avantages ressortiront mieux, à la lumière des exemples suivants, qui sont donnés à titre illustratif, et sans limitation.

### EXEMPLES

### Exemple 1 : test de ré-épithélialisation et prolifération épithéliale

### a) Matériel et méthode

Des reconstitutions d'équivalent dermique ont été préparées en mélangeant successivement dans une boîte de Petri de 6 cm de diamètre les éléments suivants :
- un milieu de culture (MEM-Dulbecco concentré 1,76 fois),
- du collagène de type I en solution dans de l'acide acétique à 0,1%, neutralisée par de la soude (5 mM),
- 10% de sérum de veau foetal délipidé, et
- 2,5 x 10⁵ fibroblastes en suspension dans du milieu MEM à 10% de sérum de veau foetal.

La formation de l'équivalent dermique a été réalisée par polymérisation en étuve à 37°C, sous une atmosphère de 5% de dioxyde de carbone.

Certaines lattices ont été pré-traitées pendant trois jours, tandis que les autres n'ont subi aucun traitement.

Le troisième jour, un fragment de 2 mm d'épithélium cutané a été implanté sur les équivalents dermiques (5 séries ont été effectuées).

Les lattices obtenues avant épidermisation et les équivalents de peau ainsi reconstitués ont été traités comme indiqué dans le Tableau 1 ci-dessous :

**Tableau 1**

| **Condition** | **Traitement dans le milieu de préparation des lattices** | **Traitement de la peau reconstituée** |
|---|---|---|
| 1 | Aucun | Aucun |
| 2 | Aucun | Composition B à 1 % (V/V) |
| 3 | Composition A à 1% (V/V) | Aucun |
| 4 | Composition A à 1% (V/V) | Composition B à 1% (V/V) |
| 5 | Composition A à 1% (V/V) | Composition A à 1% (V/V) |

La composition A était constituée de 4% en poids d'acide acexamique dans l'eau.
La composition B était constituée d'une dispersion de 30% en poids de nanocapsules, renfermant 2,5% en poids de palmitate de rétinyle, dans 70% en poids d'eau.

Le milieu a été changé tous les deux jours pendant 20 jours. Les peaux reconstruites ont été prélevées le vingtième jour pour être testées comme décrit ci-dessous.

### b) Etude histologique

Une étude histologique de l'épidermisation des peaux reconstituées a été réalisée après coloration des coupes par l'hémalun.

Le nombre de couches cellulaires au niveau de l'épithélium néo-formé (de 0 à 5 couches) a été mesuré. L'aspect fusiforme ou cubique des cellules, notamment au niveau de la couche basale, a été précisé, ainsi que la présence ou non d'une différenciation du corps muqueux.

On a ainsi mis en évidence une meilleure qualité histologique de la condition 4, par rapport aux autres conditions. Cela se traduit par la formation d'un épithélium néoformé très épais (de 1 à 5 couches cellulaires) associé à une bonne différenciation et à la présence d'une couche de cellules basales d'aspect cubique, généralement jointives.

Au contraire, les conditions 1, 3 et 5, qui n'ont pas été traitées avec la composition B, présentent des cellules le plus souvent jointives, mais d'aspect fusiformes, tandis que la condition 2 présente un nombre de couches plus faible que la condition 4.

### c) Etude immuno-histochimique

Du 5-bromo-2'-déoxyuridine (BrDU), marqueur des cellules en phase S du cycle cellulaire, a été ajouté dans le milieu de culture des peaux reconstruites entre les huitième et vingt-cinquième jours, pendant deux heures, afin d'analyser la prolifération épithéliale.

Cette analyse a été complétée par l'étude immuno-histochimique des conditions, qui consiste à analyser la prolifération après immuno-marquage à l'aide d'un anticorps anti-Ki67, afin de mesurer l'ensemble des cellules en cours de prolifération et non seulement celles en phase de synthèse de l'ADN.

Après fixation dans le formol et inclusion dans la paraffine, l'immuno-détection des cellules en phase de prolifération a été réalisée à l'aide d'une technique d'immuno-peroxydase indirecte en deux couches et révélée en DAB (kit Amersham).

Un comptage du nombre de cellules marquées au niveau de l'épithélium a été effectué. Un pourcentage de cellules en mitose a ainsi été calculé.

Les résultats sont indiqués dans le Tableau 2 ci-dessous.

**Tableau 2**

| **Condition** | **% de cellules basales** **positives** |
|---|---|
| 1 | 7,8 ± 3,05 |
| 2 | 24,2 ± 8 |
| 3 | 9,5 ± 6,3 |
| 4 | 23,3 ± 5,5 |
| 5 | 20,3 ± 4,9 |

II découle de ce tableau que la condition 4 permet l'obtention d'un taux de prolifération très satisfaisant.

### d) Conclusion

La comparaison des résultats obtenus ci-dessous pour les différentes conditions testées montre que la condition 4, qui correspond à un échantillon des peau pré-traité (avant épidermisation) par l'acide acexamique et traité (après formation de la peau reconstituée) par le palmitate de rétinyle, donne les meilleurs résultats, que ce soit en terme de modulation de la prolifération (évaluation quantitative) ou de modulation de l'épithélialisation (évaluation qualitative).

Ce test met donc en évidence l'intérêt de ce type de traitement séquentiel dans le phénomène de ré-épithélialisation.

### Exemple 2 : test de ré-épithélialisation et de prolifération épithéliale

Un test analogue à celui décrit à l'Exemple 1 a été conduit , excepté que les peaux reconstituées ont été traitées après épidermisation seulement, par introduction dans le milieu de culture, au septième jour, d'une composition constituée, en pourcentage pondéral : de 4% d'acide acexamique ; de 30% de nanocapsules renfermant 3,5% en poids de palmitate de rétinyle ; de 0,075% de conservateur et de 0,075% d'anti-oxydant dans de l'eau.

L'analyse histologique montre qu'au vingt-cinquième jour, les cinq séries d'échantillons réalisées présentent toutes un néo-épithélium formé d'une couche basale et de une à quatre couches de corps muqueux. Par comparaison, l'épithélium néo-formé des mêmes peaux reconstituées non traitées ne comporte qu'une couche basale et apparaît avec cinq à dix jours de retard par rapport aux séries traitées.

L'analyse immuno-histochimique révèle une division cellulaire importante, correspondant à la prolifération des cellules, tandis que la phase de prolifération cellulaire des peaux reconstruites non traitées apparaît, là encore, avec retard.

Ce test met donc en évidence, par rapport au témoin non traité, une modulation positive de la ré-épithélialisation par l'association de l'acide acexamique avec le palmitate de rétinyle, accompagnée d'une diminution du temps de reconstitution des épithéliums corrélée à une augmentation du nombre de cellules épithéliales en mitose.

### Exemple 3 : composition cosmétique

| | |
|---|---|
| Octyldodécanol | 4 % |
| Sulfate de magnésium | 0,7 % |
| Huile minérale | 18 % |
| Conservateurs | 0,75 % |
| Glycérine | 7 % |
| Cetyl diméthicone copolyol | 2 % |
| Nanocapsules de palmitate de rétinyle (2,5% de matière active) | 0,3 % |
| Acide acexamique | 0,04% |
| Eau qsp. | 100 % |

Cette crème peut appliquée sur les mains aussi souvent que nécessaire pour apaiser les rougeurs et tiraillements et refermer les gerçures.

### Exemple 4 : composition cosmétique

On prépare, de manière classique, pour l'homme du métier, la composition suivante :

| | |
|---|---|
| Octyldodécanol | 4 % |
| Sulfate de magnésium | 0,7 % |
| Huile minérale | 18 % |
| Conservateurs | 0,75 % |
| Glycérine | 7 % |
| Cetyl diméthicone copolyol | 2 % |
| Nanocapsules de palmitate de rétinyle (2,5% de matière active) | 0,3 % |
| Lycopène | 0,0013% |
| Acide acexamique | 0,04 % |
| Eau qsp. | 100 % |

### Exemple 5 : préparations pour administration séquentielle

On prépare, de manière classique, pour l'homme du métier, les compositions A et B suivantes.

| Composition A : crème pour la peau | |
|---|---|
| Octyldodécanol | 4 % |
| Sulfate de magnésium | 0,7 % |
| Huile minérale | 18 % |
| Conservateurs | 0,75 % |
| Glycérine | 7 % |
| Cetyl diméthicone copolyol | 2 % |
| Nanocapsules de palmitate de rétinyle (2,5% de matière active) | 0,3 % |
| Acide acexamique | 0,04 % |
| Eau qsp. | 100 % |

| Composition B : gélules | |
|---|---|
| Beta-carotène | 5 mg |
| Lycopène | 2,6 mg |
| Maltodextrine | 300 mg |

La composition pour la peau est destinée à être appliquée le matin sur les parties exposées du corps, tandis que les gélules seront ingérées en fin de journée. Ce protocole sera répété pendant plusieurs jours voire semaines.

### Exemple 6 : préparations pour administration séquentielle

On prépare, de manière classique, pour l'homme du métier, les compositions A et B suivantes.

| Composition A : crème pour la peau | |
|---|---|
| Octyldodécanol | 4 % |
| Sulfate de magnésium | 0,7 % |
| Huile minérale | 18 % |
| Conservateurs | 0,75 % |
| Glycérine | 7 % |
| Cetyl diméthicone copolyol | 2 % |
| Nanocapsules de palmitate de rétinyle (2,5% de matière active) | 0,3 % |
| Acide acexamique | 0,04 % |
| Eau qsp. | 100 % |

| Composition B : complément nutritionnel - capsules molles | |
|---|---|
| Beta-carotène | 5 mg |
| Lycopène | 2,6 mg |
| Huile de soja hydrogénée | 40 mg |
| Huile de blé | 95 mg |
| Lécithine de soja | 20 mg |
| Tocophérols naturels | 5 mg |
| Acide ascorbique | 30 mg |

La composition pour la peau ci-dessus est destinée à être appliquée sur les parties exposées du corps sensiblement simultanément à l'ingestion du complément nutritionnel, dont l'utilisation pourra être prolongée pendant quelques jours. La cinétique d'absorption des nutriments étant plus lente que l'absorption percutanée de l'acide acexamique, on obtiendra ainsi un effet séquentiel de l'acide acexamique et des caroténoïdes qui permet d'offrir un bénéfice maximal à la peau.

## Revendications

**1.** Utilisation cosmétique d'une composition comprenant, dans un milieu physiologiquement acceptable, de 0,001% à 1% en poids d'acide acéxamique et/ou l'un de ses sels, et de 0,00001% à 0,1% en poids d'au moins un composé choisi parmi les rétinoïdes et les caroténoïdes pour prévenir ou lutter contre les gerçures, les tiraillements et/ou les rougeurs.

**2.** Utilisation selon la revendication 1, **caractérisée en ce que** ledit rétinoïde est choisi parmi : le rétinol, le rétinal, l'acide rétinoïque 13-cis, l'acide rétinoïque all-trans et les esters de rétinyle.

**3.** Utilisation selon la revendication 2, **caractérisée en ce que** lesdits esters de rétinyle sont choisis parmi l'acétate de rétinyle, le propionate de rétinyle et le palmitate de rétinyle.

**4.** Utilisation selon l'une quelconque des revendications 1 à 3, **caractérisée en ce que** le caroténoïde est choisi parmi l'α-carotène, le β-carotène, le lycopène, la zéaxanthine, la lutéine, l'ataxanthine, la canthaxanthine et la cryptoxanthine.

**5.** Utilisation selon l'une quelconque des revendications 1 à 4, **caractérisée en ce que** le rétinoïde et/ou le caroténoïde sont encapsulés.

**6.** Utilisation selon l'une quelconque des revendication 1 à 5, **caractérisée en ce que** la composition renferme l'acide acexamique libre.

**7.** Utilisation selon la revendication 6, **caractérisée en ce que** lesdits sels d'acide acexamique sont choisis parmi les sels de métaux alcalino-terreux et les sels de métaux de transition de l'acide acexamique.

**8.** Utilisation selon la revendication 7, **caractérisée en ce que** lesdits sels d'acide acexamique sont choisis parmi les sels de zinc, de calcium et/ou de magnésium d'acide acexamique.

**9.** Utilisation selon l'une quelconque des revendication 1 à 8, **caractérisée en ce que** la composition comprend de 0,01 % à 0,1% en poids d'acide acexamique et/ou de son sel, par rapport au poids total de la composition.

**10.** Utilisation selon l'une quelconque des revendications 1 à 9, **caractérisée en ce que** la composition comprend de 0,1% à 0,001% en poids de rétinoïde ou caroténoïde, par rapport au poids total de la composition.

**11.** Utilisation selon l'une quelconque des revendication 1 à 10, **caractérisée en ce que** la composition est adaptée à une application topique sur la peau.

**12.** Utilisation selon l'une quelconque des revendication 1 à 10, **caractérisée en ce que** la composition est sous la forme d'une émulsion eau-dans-huile.

**13.** Utilisation selon l'une quelconque des revendications 1 à 12, **caractérisée en ce que** la composition est appliquée sur les mains.

**16.** Utilisation selon l'une quelconque des revendications 1 à 12, **caractérisée en ce que** la composition est appliquée sur les lèvres.

**17.** Utilisation d'au moins un premier composé choisi parmi l'acide acexamique et ses sels, et d'au moins un second composé choisi parmi les rétinoïdes et caroténoïdes, pour fabriquer respectivement une première et une seconde préparations dermatologiques destinées à être administrées successivement pour favoriser la ré-épithélialisation de la peau ou des muqueuses.

**18.** Utilisation selon la revendication 17, **caractérisée en ce que** lesdites première et seconde préparations sont destinées à favoriser la cicatrisation des plaies ou brûlures.

**19.** Utilisation selon la revendication 17 ou 18, **caractérisée en ce que** ladite première préparation est appliquée topiquement sur la peau ou les muqueuses et ladite seconde préparation est administrée par voie orale ou par injection.

**20.** Utilisation selon la revendication 17 ou 18, **caractérisée en ce que** ladite première préparation et ladite seconde préparation sont appliquées topiquement sur la peau ou les muqueuses.

**21.** Procédé de fabrication d'une peau reconstruite ou artificielle, comprenant les étapes successives (a) d'application sur un équivalent dermique d'une première composition comprenant au moins un premier composé choisi parmi l'acide acexamique et ses sels, (b) d'application sur ledit équivalent dermique d'une seconde composition comprenant au moins un second composé choisi parmi les rétinoïdes et les caroténoïdes.
